# EUROPÄISCHE PATENTSCHRIFT

(11) **EP 0 700 904 B1**
(45) Veröffentlichungstag und Bekanntmachung des Hinweises auf die Patenterteilung: **05.12.2001**
(21) Anmeldenummer: 95113845.2
(22) Anmeldetag: 04.09.1995
(51) Int. Cl.: C07D 213/89, C07D 215/60, A61K 31/44, A61K 31/47

(54) **Mit Heterocyclen-N-Oxid-substituierte Benzoylguanidine, Verfahren zu ihrer Herstellung, ihre Verwendung als Medikament oder Diagnostikum sowie sie enthaltendes Medikament**
Heterocyclic-N-oxide substituted benzoylguanidines, process for their preparation, their use as medicament or diagnostic as well as medicaments containing them
Benzoylguanidines substitués avec des N-oxides hétérocycliques, procédé pour leur préparation, leur utilisation comme médicament ou diagnostic et médicaments les contenant

(30) Priorität: 09.09.1994 DE 4432106
(43) Veröffentlichungstag der Anmeldung: 13.03.1996
(73) Patentinhaber: HOECHST AKTIENGESELLSCHAFT, 65929 Frankfurt am Main (DE)
(72) Erfinder: Kleemann, Heinz-Werner, Dr., D-65474 Bischofsheim (DE); Lang, Hans-Jochen, Dr., D-65719 Hofheim (DE); Schwark, Jan-Robert, Dr., D-65779 Kelkheim (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 165 904
- EP-A- 0 405 487
- EP-A- 0 602 523
- DD-A- 271 903
- FR-A- 2 207 917
- US-A- 3 931 181
- US-A- 5 212 182
- CHEMICAL ABSTRACTS, vol. 77, no. 9, 1972 Columbus, Ohio, US; abstract no. 61765q, & ITSUU KENKYUSHO NEMPO , Nr. 16, 1971 Seiten 25-39, M. NATSUME ET AL & DATABASE REGISTRY CHEMICAL ABSTRACTS SERVICE, COLUMBUS, OHIO, US (STN),
- CHEMICAL ABSTRACTS, vol. 84, no. 19, 1976 Columbus, Ohio, US; abstract no. 135477r, & JP-A-50 111 076 (SHIONOGI AND CO LTD) & DATABASE REGISTRY CHEMICAL ABSTRACTS SERVICE, COLUMBUS, OHIO, US (STN),
- CHEMICAL ABSTRACTS, vol. 59, no. 4, 1963 Columbus, Ohio, US; abstract no. 4492e, & WISS. Z. TECH. HOCHSCH. CHEM. LEUNA-MERSEBURG, Bd. 4, Nr. 3/4, 1962 Seiten 213-218, G. BUCHMANN & DATABASE REGISTRY CHEMICAL ABSTRACTS SERVICE, COLUMBUS, OHIO, US (STN) ,

## Beschreibung

Die Erfindung betrifft Benzoylguanidine der Formel I 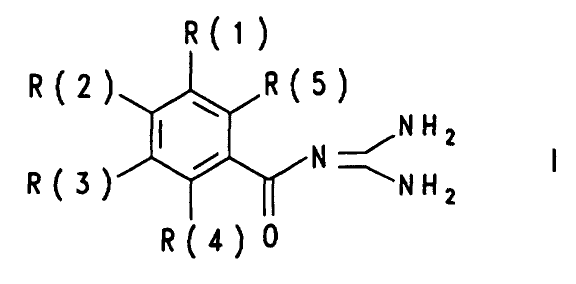 worin bedeuten:
- R(1): (C₁-C₄)-Alkyl, (C₂-C₄)-Alkenyl oder -CₘH₂ₘR(14),
m Null, 1 oder 2;
R(14) (C₅-C₆)-Cycloalkyl oder Phenyl,
welches nicht substituiert oder substituiert ist mit 1 - 2 Substituenten ausgewählt aus der Gruppe bestehend aus F, Cl, CF₃, Methyl, Methoxy und NR(15)R(16);
R(15) und R(16)
Wasserstoff oder CH₃;
oder
- R(1): Wasserstoff, F, Cl, Br, I, -C≡N, R(22)-SO₂, R(23)R(24)N-CO, R(25)-CO-oder R(26)R(27)N-SO₂-;
R(22), R(23), R(25) und R(26)
unabhängig voneinander (C₁-C₄)-Alkyl, (C₂-C₄)-Alkenyl,
-CₙH₂ₙ-R(29) oder CF₃;
n Null, 1 oder 2;
R(29) (C₅-C₆)-Cycloalkyl oder Phenyl,
welches nicht substituiert ist oder substituiert mit 1 - 2 Substituenten ausgewählt aus der Gruppe bestehend aus F, Cl, CF₃, Methyl, Methoxy und NR(30)R(31);
R(30) und R(31)
Wasserstoff oder Methyl;
oder
R(23), R(25) und R(26)
Wasserstoff;
R(24) und R(27)
unabhängig voneinander Wasserstoff oder Methyl;
oder R(23) und R(24) sowie R(26) und R(27)
gemeinsam 4 oder 5 Methylengruppen, von denen eine CH₂-Gruppe durch Sauerstoff, S, NH, N-CH₃ oder N-Benzyl ersetzt sein kann;
oder
- R(1): OR(35) oder NR(35)R(36);
R(35) und R(36)
unabhängig voneinander Wasserstoff, (C₁-C₄)-Alkyl;
oder R(35) und R(36)
gemeinsam 4 - 5 Methylengruppen, von denen eine CH₂-Gruppe durch Sauerstoff, S, NH, N-CH₃ oder N-Benzyl ersetzt sein kann;
einer der Substituenten R(2) und R(3)
Imidazolyl-N-Oxid, Pyrazolyl-N-Oxid, Triazolyl-N-Oxid, Tetrazolyl-N-Oxid, Oxazolyl-N-Oxid, Isoxazolyl-N-Oxid, Thiazolyl-N-Oxid, Isothiazolyl-N-Oxid, Pyridyl-N-Oxid, Pyrazinyl-N-Oxid, Pyrimidinyl-N-Oxid, Pyridazinyl-N-Oxid, Indazolyl-N-Oxid, Chinolyl-N-Oxid, Isochinolyl-N-Oxid, Phthalazinyl-N-Oxid, Chinoxalinyl-N-Oxid, Chinazolinyl-N-Oxid, Cinnolinyl-N-Oxid,
das über C oder N verknüpft ist und das unsubstituiert oder substituiert ist mit 1 - 2 Substituenten ausgewählt aus der Gruppe bestehend aus F, Cl, CF₃, CH₃, Methoxy, Hydroxy, Amino, Methylamino und Dimethylamino;
oder einer der Substituenten R(2) und R(3)
-SR(10), -OR(10), -NR(10)R(11) oder -CR(10)R(11)R(12);
R(10) -CₐH₂ₐ-[Imidazolyl-N-Oxid, Pyrazolyl-N-Oxid, Triazolyl-N-Oxid,
Tetrazolyl-N-Oxid, Oxazolyl-N-Oxid, Isoxazolyl-N-Oxid, Thiazolyl-N-Oxid, Isothiazolyl-N-Oxid, Pyridyl-N-Oxid, Pyrazinyl-N-Oxid, Pyrimidinyl-N-Oxid, Pyridazinyl-N-Oxid, Indazolyl-N-Oxid, Chinolyl-N-Oxid, Isochinolyl-N-Oxid, Phthalazinyl-N-Oxid, Chinoxalinyl-N-Oxid, Chinazolinyl-N-Oxid, Cinnolinyl-N-Oxid],
das über C oder N verknüpft ist und das unsubstituiert oder substituiert ist mit 1 - 2 Substituenten ausgewählt aus der Gruppe bestehend aus F, Cl, CF₃, CH₃, Methoxy, Hydroxy, Amino, Methylamino und Dimethylamino;
a Null oder 1;
R(11), R(12)
Wasserstoff oder Methyl;
und der jeweils andere Substituent R(2) und R(3)
unabhängig voneinander (C₁-C₄)-Alkyl, Wasserstoff, F, Cl, Br oder I;
R(4) und R(5)
unabhängig voneinander Wasserstoff, Methyl, F, Cl, -OR(32),
-NR(33)R(34) oder CF₃;
R(32), R(33) und R(34)
unabhängig voneinander Wasserstoff oder Methyl;
sowie deren pharmazeutisch verträgliche Salze.

Bevorzugt sind Verbindungen der Formel I in denen bedeuten:
- R(1): (C₁-C₄)-Alkyl, (C₂-C₄)-Alkenyl oder -CₘH₂ₘR(14);
m Null, 1 oder 2;
R(14) (C₅-C₆)-Cycloalkyl oder Phenyl,
welches nicht substituiert oder substituiert ist mit 1 - 2 Substituenten ausgewählt aus der Gruppe bestehend aus F, Cl, CF₃, Methyl, Methoxy und NR(15)R(16);
R(15) und R(16)
Wasserstoff oder CH₃;
oder
- R(1): Wasserstoff, F, Cl, Br, I, -C≡N, R(22)-SO₂, R(23)R(24)N-CO, R(25)-CO-oder R(26)R(27)N-SO₂-;
R(22), R(23), R(25) und R(26)
unabhängig Methyl oder CF₃;
oder
R(23), R(25) und R(26)
Wasserstoff;
R(24) und R(27)
unabhängig voneinander Wasserstoff oder Methyl;
oder
- R(1): OR(35) oder NR(35)R(36);
R(35) und R(361
unabhängig voneinander Wasserstoff, (C₁-C₄)-Alkyl;
oder
R(35) und R(36)
gemeinsam 4 - 5 Methylengruppen, von denen eine CH₂-Gruppe durch Sauerstoff, S, NH, N-CH₃ oder N-Benzyl ersetzt sein kann,
einer der Substituenten R(2) und R(3)
Imidazolyl-N-Oxid, Pyrazolyl-N-Oxid, Triazolyl-N-Oxid, Tetrazolyl-N-Oxid, Oxazolyl-N-Oxid, Isoxazolyl-N-Oxid, Thiazolyl-N-Oxid, Isothiazolyl-N-Oxid, Pyridyl-N-Oxid, Pyrazinyl-N-Oxid, Pyrimidinyl-N-Oxid, Pyridazinyl-N-Oxid, Indazolyl-N-Oxid, Chinolyl-N-Oxid, Isochinolyl-N-Oxid, Phthalazinyl-N-Oxid, Chinoxalinyl-N-Oxid, Chinazolinyl-N-Oxid, Cinnolinyl-N-Oxid,
das über C oder N verknüpft ist und das unsubstituiert oder substituiert ist mit 1 - 2 Substituenten ausgewählt aus der Gruppe bestehend aus F, Cl, CF₃, CH₃, Methoxy, Hydroxy, Amino, Methylamino und Dimethylamino;
oder
einer der Substituenten R(2) und R(3)
-SR(10), -OR(10), -NR(10)R(11) oder -CR(10)R(11)R(12);
R(10) Imidazolyl-N-Oxid, Pyrazolyl-N-Oxid, Triazolyl-N-Oxid, Tetrazolyl-N-Oxid, Oxazolyl-N-Oxid, Isoxazolyl-N-Oxid, Thiazolyl-N-Oxid, Isothiazolyl-N-Oxid, Pyridyl-N-Oxid, Pyrazinyl-N-Oxid, Pyrimidinyl-N-Oxid, Pyridazinyl-N-Oxid, Indazolyl-N-Oxid, Chinolyl-N-Oxid, Isochinolyl-N-Oxid, Phthalazinyl-N-Oxid, Chinoxalinyl-N-Oxid, Chinazolinyl-N-Oxid, Cinnolinyl-N-Oxid,
das über C oder N verknüpft ist und das unsubstituiert oder substituiert ist mit 1 - 2 Substituenten ausgewählt aus der Gruppe bestehend aus F, Cl, CF₃, CH₃, Methoxy, Hydroxy, Amino, Methylamino und Dimethylamino;
R(11) und R(12)
Wasserstoff oder Methyl;
und der jeweils andere Substituent R(2) oder R(3)
unabhängig voneinander (C₁-C₄)-Alkyl, Wasserstoff, F, Cl, Br oder I;
R(4) und R(5)
unabhängig voneinander Wasserstoff, Methyl, F, Cl, -OR(32),-NR(33)R(34) oder CF₃;
R(32), R(33) und R(34)
unabhängig voneinander Wasserstoff oder Methyl;
sowie deren pharmazeutisch verträgliche Salze.

Als Heteroaryl-N-Oxid gelten Imidazolyl-N-Oxid, Pyrazolyl-N-Oxid, Triazolyl-N-Oxid, Tetrazolyl-N-Oxid, Oxazolyl-N-Oxid, Isoxazolyl-N-Oxid, Thiazolyl-N-Oxid, Isothiazolyl-N-Oxid, Pyridyl-N-Oxid, Pyrazinyl-N-Oxid, Pyrimidinyl-N-Oxid, Pyridazinyl-N-Oxid, Indazolyl-N-Oxid, Chinolyl-N-Oxid, Isochinolyl-N-Oxid, Phthalazinyl-N-Oxid, Chinoxalinyl-N-Oxid, Chinazolinyl-N-Oxid, Cinnolinyl-N-Oxid.

Enthält einer der Substituenten R(1) bis R(5) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkyl- und Perfluoralkylreste können sowohl geradkettig wie verzweigt vorliegen.

Die Erfindung betrifft weiterhin ein Verfahren zur Herstellung der Verbindungen der Formel I, dadurch gekennzeichnet, daß man
eine Verbindung der Formel II 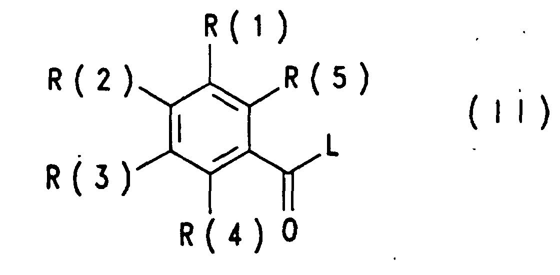 mit Guanidin umsetzt, worin L für eine leicht nucleophil substituierbare Fluchtgruppe steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäure-chloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.
Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol (L = 1-Imidazolyl, Staab, Angew. Chem. Int. Ed. Engl. 1,351-367 (1962)), die gemischten Anhydride II mit Cl-COOC₂H₅ oder Tosylchlorid in Gegenwart von Triethylamin in einem inerten Lösungsmittel, wie auch die Aktivierungen von Benzoesäuresäuren mit Dicyclohexylcarbodiimid (DCC) oder mit O-[(Cyano(ethoxycarbonyl)-methylen)amino]-1,1,3,3-tetramethyluronium-tetrafluoroborat ("TOTU")[Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der allgemeinen Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF zwischen 20°C und Siedetemperatur dieser Lösungsmittel bewährt. Bei den meisten Umsetzungen von Verbindungen II mit salzfreien Guanidin wurde vorteilhaft in inerten Lösungsmitteln wie THF, Dimethoxyethan, Dioxan oder Isopropanol gearbeitet. Aber auch Wasser kann als Lösungsmittel dienen.

Wenn L = Cl bedeutet, arbeitet man vorteilhaft unter Zusatz eines Säurefängers, z.B. in Form von überschüssigen Guanidin zur Abbindung der Halogenwasserstoffsäure.

Die unbekannten Verbindungen der Formel II können nach literatur-bekannten Methoden hergestellt werden, indem man beispielsweise 4-Halogen-3-chlorsulfonylbenzoesäuren mit Ammoniak oder Aminen in 3-Aminosulfonyl-4-Halogen-Benzoesäuren bzw. mit einem schwachen Reduktionsmittel wie Natriumbisulfit und anschließender Alkylierung in 3-Alkylsulfonyl-4-Halogen-Benzoesäuren überführt und nach einer der oben beschriebenen Verfahrensvarianten zu erfindungsgemäßen Verbindungen I umgesetzt werden.

Die Einführung von substituierten Schwefel- Sauerstoff- oder Stickstoffnucleophilen gelingt durch literaturbekannte Methoden der nucleophilen Substitution am Aromaten. Als Abgangsgruppe haben sich bei dieser Substitution Halogenide und Trifluormethansulfonate bewährt. Man arbeitet vorteilhaft in einem dipolar aprotischen Lösungsmittel, wie zum Beispiel DMF oder TMU bei einer Temperatur zwischen 0°C und dem Siedepunkt des Lösungsmittels, bevorzugt zwischen 80°C und dem Siedepunkt des Lösungsmittels. Als Säurefänger dient vorteilhaft ein Alkali- oder Erdalkalisalz mit einem Anion hoher Basizität und geringer Nucleophilie, wie zum Beispiel K₂CO₃ oder CsCO₃.

Die Einführung der Alkyl- oder Arylsubstituenten gelingt durch literaturbekannte Methoden des Palladium-vermittelten cross-couplings von Arylhalogeniden mit beispielsweise Organozinkverbindungen, Organostannanen, Organoboronsäuren oder Organoboranen.

Die Oxidation des Stickstoffs im Heteroaryl-Substituenten gelingt an geeigneten Zwischenverbindungen wie dem Benzoesäureester mit im Prinzip bekannten Methoden. Bewährt hat sich beispielsweise m-Chlorperbenzoesäure in einem inerten Lösungsmittel wie Methylenchlorid bei einer Temperatur zwischen -30°C und dem Siedepunkt des Lösungsmittels.

Zwischenprodukte der Formel III werden zum Herstellen der Verbindungen I benutzt, welche Zwischenprodukte der Formel III bereits wesentliche Teile der Struktur der Verbindungen I enthalten: 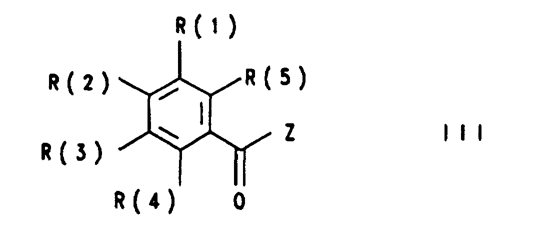 mit
- Z: Cl, 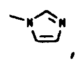 OR(45), OMet oder OH;
R(45) (C₁-C₈)-Alkyl oder (C₁-C₄)-Alkylen-Phenyl;
Met Ion eines Alkali- oder Erdalkali-Metalls.

Dabei handelt es sich um die den Guanidinen entsprechenden Säureester, die Salze und die freien Säuren, welche zu den Guanidinen umgesetzt werden.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 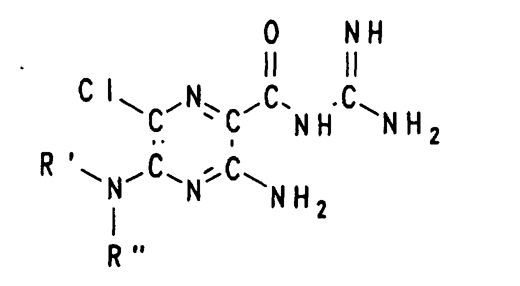 Amilorid: R',R" = H
Dimethylamilorid: R',R" = CH₃
Ethylisopropylamilorid: R' = C₂H₅, R" = CH(CH₃)₂

Darüberhinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl.1): 167 (1988) (book of abstracts)]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamitorid.

In der US-Patentschrift 5 091 394 (HOE 89/F 288) sind Benzoylguanidine beschrieben, die in der dem Rest R(1) entsprechenden Stellung ein Wasserstoff-Atom tragen. In der deutschen Patentanmeldung P 42 04 575.4 (US 5 373 024) werden Benzoylguanidine vorgeschlagen, in welchen aber die Substituenten nicht die nach der vorliegenden Erfindung beanspruchten Bedeutungen haben.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Die EP-A 602 523 beschreibt verwandte Verbindungen, jedoch ist darin keine Verbindungen mit einer N-Oxid-Struktur beschrieben oder nahegelegt.

Es war daher überraschend, daß die erfindungsgemäßen Verbindungen keine unerwünschten und nachteiligen salidiuretischen, jedoch sehr gute antiarrhythmische Eigenschaften aufweisen, die bei der Behandlung von Krankheiten wichtig sind, wie sie beispielsweise bei Sauerstoff-Mangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na ⁺/H ⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei OrganTransplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des Zentralnervensystems, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na ⁺/H ⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den bekannten Verbindungen weisen die Verbindungen nach der Erfindung eine signifikant verbesserte Wasserlöslichkeit auf. Daher sind sie wesentlich besser für i.V.-Applikationen geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- TMU: N,N,N',N'-Tetramethylharnstoff
- El: electron impact
- DCI: Desorption-Chemical Ionisation
- RT: Raumtemperatur
- EE: Ethylacetat (EtOAc)
- DIP: Diisopropylether
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan
- THF: Tetrahydrofuran
- eq: Äquivalent
- ES: Elektrospray-lonisation
- Me: Methyl
- Et: Ethyl
- Bn: Benzyl
- ZNS: Zentralnervensystem
- Brine: gesättigte wäßrige NaCl-Lösung
- Met: Metall

### Experimenteller Teil

### Allgemeine Vorschrift zur Herstellung von Benzoyl-guanidinen (I)

### Variante A: aus Benzoesäuren (II, L = OH)

0,01 M des Benzoesäurederivates der Formel II löst bzw. suspendiert man in 60 ml wasserfreiem THF und versetzt sodann mit 1,78 g (0,011 M) Carbonyldiimidazol. Nach dem Rühren über 2 Stunden bei RT werden 2,95 g (0,05 M) Guanidin in die Reaktionslösung eingetragen. Nach dem Rühren über Nacht destilliert man das THF unter vermindertem Druck (im Rotationsverdampfer) ab, versetzt mit Wasser, stellt mit 2N HCI auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I)

### Variante B: aus Benzoesäure-alkylestern (II, L = O-Alkyl)

5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert, in 300 ml EE aufgenommen und 3 x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.

### (Salzbildung vergleiche Variante A)

### Beispiel 1

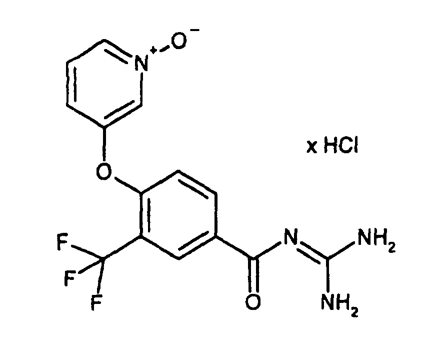

### 4-(Pyridin-N-oxid-3-yloxy)-3-trifluormethyl-benzoylguanidin, Hydrochlorid

500 mg 4-(Pyridin-N-oxid-3-yloxy)-3-trifluormethyl-benzoesäuremethylester und 472 mg Guanidin werden nach der allgemeinen Vorschrift Variante B in 5 ml Isopropanol guanyliert. Man erhält 250 mg farbloses Öl. mp (Hydrochlorid) = 180 °C.
R_{f} (EE/MeOH 1:1) = 0.27 MS (ES) : 341 (M+H)⁺
a) 4-(Pyridin-N-oxid-3-yloxy)-3-trifluormethyl-benzoesäuremethylester
   1.1 g 4-(3-Pyridyloxy)-3-trifluoromethyl-benzoesäuremethylester und 912 mg 3-Chlorperbenzoesäure werden in 10 ml CH₂Cl₂ gelöst und 18 h bei RT gerührt. Anschließend werden 126 mg Na₂SO₃ zugegeben und das Solvens im Vakuum entfernt. In 100 ml EE wird aufgenommen und 1 x mit 100 ml gesättigter wäßriger Na₂SO₃-Lösung und 3 x mit je 100 ml gesättigter wäßriger Na₂CO₃-Lösung extrahiert. Über Na₂SO₄ wird getrocknet und das Solvens im Vakuum entfernt. Man erhält 1.1 g farbloses Öl.
   R_{f} (EE/MeOH 10 : 1) = 0.19 MS (ES) : 314 (M + H)⁺
b) 4-(3-Pyridyloxy)-3-trifluoromethyl-benzoesäuremethylester
   2 mmol 4-Fluor-3-trifluoromethyl-benzoesäuremethylester, 2 mmol 3-Hydroxypyridin und 4 mmol K₂CO₃ werden in 15 ml DMF (wasserfrei) 1.5 h bei 110 °C gerührt. Das Gemisch wird anschließend auf 100 ml Wasser gegossen und 3 x mit je 50 ml EE extrahiert. Über Na₂SO₄ wird getrocknet, das Lösungsmittel im Vakuum entfernt und das Produkt ohne weitere Reinigung weiter umgesetzt. 500 mg farbloses Öl.
   R_{f}(MTB) = 0.33 MS (ES) : 298 (M + 1)

### Beispiel 2

### 4-(Chinaldin-N-oxid-6-yloxy)-3-methylsulfonyl-benzoylguanidin, Hydrochlorid

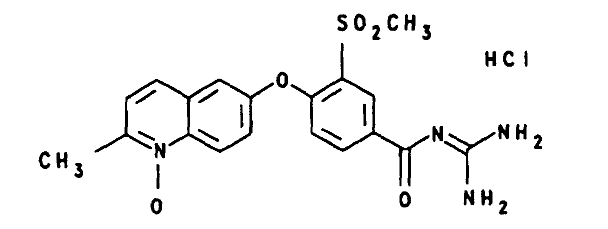

400 mg 4-(Chinaldin-N-oxid-6-yloxy)-3-methylsulfonyl-benzoesäuremethylester und 300 mg Guanidin werden nach der allgemeinen Vorschrift, Variante B in 15 ml Isopropanol guanyliert.
Man erhält 140 mg eines glasigen Feststoffs.
mp (Hydrochlorid) = 247°C.
R_{f} (EE/MeOH 5:1) = 0.06 MS (ES) : 417 (M + H)⁺
a) 4-(Chinaldin-N-oxid-6-yloxy)-3-methylsulfonyl-benzoesäuremethylester 372 mg 4-(6-Chinaldinyloxy)-3-methylsulfonyl-benzoesäuremethylester werden analog Beispiel 1 a) zum N-oxidiert und ohne Reinigung weiter umgesetzt.
b) 4-(6-Chinaldinyloxy)-3-methylsulfonyl-benzoesäuremethylester
   2 g 6-Hydroxychinaldin, 3,12 g 4-Chlor-3-methylsulfonylbenzoesäuremethylester und 12,28 g Cs₂CO₃ werden in 50 ml wasserfreiem Tetramethylharnstoff 1 h bei 110 °C gerührt. Nach dem Abkühlen werden 250 ml gesättigte wäßrige NaHCO₃-Lösung zugegeben und 3 x mit 125 ml EE extrahiert. Über Na₂SO₄ wird getrocknet, das Lösungsmittel im Vakuum entfernt und mit MTB chromatographiert. Man erhält 3,98 g eines farblosen Schaums.
   R_{f} (MTB) = 0.31 MS (ES) : 372 (M+H)⁺

Die Verbindung des Beispiels 3 wurde analog Beispiel 2 synthetisiert:

### Beispiel 3

### 4-(Chinaldin-N-oxid-6-yloxy)-3-trifluormethyl-benzoylguanidin, Hydrochlorid

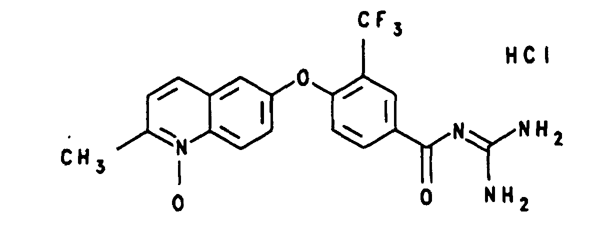

R_{f} (EE/MeOH 5:1) = 0.11 MS (ES) : 441 (M+H)⁺

### Pharmakologische Daten

### Inhibition des Na ⁺ /H ⁺ -Exchangers von Kaninchenerythrocyten

Weiße Neuseeland-Kaninchen (Ivanovas) erhielten eine Standard-Diät mit 2% Cholesterin für sechs Wochen, um den Na⁺/H ⁺-Austausch zu aktivieren und so den Na ⁺ -Influx in die Erythrocyten via Na ⁺ /H ⁺ -Austausch den Na⁺-Influx in die Erythrocyten via Na ⁺ /H ⁺ -Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 *µ*l dienten zur Messung des Na⁺-Ausgangsgehalts der Erythrocyten.

Um den Amilorid-sensitiven Natrium-Influx zu bestimmen, wurden 100 *µ*l jeder Blutprobe in jeweils 5 ml eines hyperosmolaren Salz-Sucrose-Mediums (mmol/l: 140 NaCI, 3 KCI, 150 Sucrose, 0,1 Ouabain, 20 Tris-hydroxymethylaminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

| Inhibition des Na⁺/H⁺-Exchangers: | |
|---|---|
| Beispiel | IC₅₀ µmol/l |
| 1 | 0.08 |
| 2 | 0.3 |
| 3 | 0.019 |

## Patentansprüche

1. Benzoylguanidine der Formel I 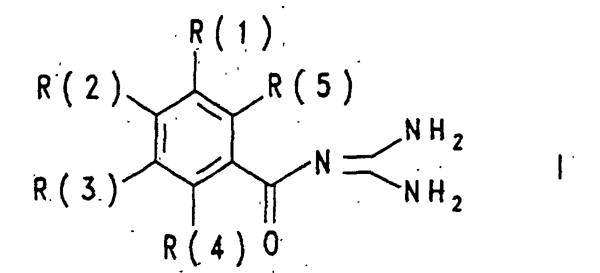 worin bedeuten:
R(1) (C₁-C₄)-Alkyl, (C₂-C₄)-Alkenyl oder -CₘH₂ₘR(14),
m Null, 1 oder 2;
R(14) (C₅-C₆)-Cycloalkyl oder Phenyl,
welches nicht substituiert oder substituiert ist mit 1 - 2 Substituenten ausgewählt aus der Gruppe bestehend aus F, Cl, CF₃, Methyl, Methoxy und NR(15)R(16);
R(15) und R(16)
Wasserstoff oder CH₃;
oder
R(1) Wasserstoff, F, Cl, Br, I, -C≡N, R(22)-SO₂, R(23)R(24)N-CO, R(25)-CO-oder R(26)R(27)N-SO₂-;
R(22), R(23), R(25) und R(26)
unabhängig (C₁-C₄)-Alkyl, (C₂-C₄)-Alkenyl, -CₙH₂ₙ-R(29) oder CF₃;
n Null, 1 oder 2;
R(29) (C₅-C₆)-Cycloalkyl oder Phenyl,
welches nicht substituiert ist oder substituiert mit 1 - 2 Substituenten ausgewählt aus der Gruppe bestehend aus F, Cl, CF₃, Methyl, Methoxy und NR(30)R(31);
R(30) und R(31)
Wasserstoff oder Methyl;
oder
R(23), R(25) und R(26)
Wasserstoff;
R(24) und R(27)
unabhängig voneinander Wasserstoff oder Methyl;
oder R(23) und R(24) sowie R(26) und R(27)
gemeinsam 4 oder 5 Methylengruppen, von denen eine CH₂-Gruppe durch Sauerstoff, S, NH, N-CH₃ oder N-Benzyl ersetzt sein kann;
oder
R(1) OR(35) oder NR(35)R(36);
R(35) und R(36)
unabhängig voneinander Wasserstoff, (C₁-C₄)-Alkyl;
oder R(35) und R(36)
gemeinsam 4 - 5 Methylengruppen, von denen eine CH₂-Gruppe
durch Sauerstoff, S, NH, N-CH₃ oder N-Benzyl ersetzt sein kann;
einer der Substituenten R(2) und R(3)
Imidazolyl-N-Oxid, Pyrazolyl-N-Oxid, Triazolyl-N-Oxid, Tetrazolyl-N-Oxid, Oxazolyl-N-Oxid, Isoxazolyl-N-Oxid, Thiazolyl-N-Oxid, Isothiazolyl-N-Oxid, Pyridyl-N-Oxid, Pyrazinyl-N-Oxid, Pyrimidinyl-N-Oxid, Pyridazinyl-N-Oxid, Indazolyl-N-Oxid, Chinolyl-N-Oxid, Isochinolyl-N-Oxid, Phthalazinyl-N-Oxid, Chinoxalinyl-N-Oxid, Chinazolinyl-N-Oxid, Cinnolinyl-N-Oxid,
das über C oder N verknüpft ist und das unsubstituiert oder substituiert ist mit 1 - 2 Substituenten ausgewählt aus der Gruppe bestehend aus F, Cl, CF₃, CH₃, Methoxy, Hydroxy, Amino, Methylamino und Dimethylamino;
oder einer der Substituenten R(2) und R(3)
-SR(10), -OR(10), -NR(10)R(11) oder -CR(10)R(11)R(12);
R(10) -CₐH₂ₐ-[Imidazolyl-N-Oxid, Pyrazolyl-N-Oxid, Triazolyl-N-Oxid,
Tetrazolyl-N-Oxid, Oxazolyl-N-Oxid, Isoxazolyl-N-Oxid, Thiazolyl-N-Oxid, Isothiazolyl-N-Oxid, Pyridyl-N-Oxid, Pyrazinyl-N-Oxid, Pyrimidinyl-N-Oxid, Pyridazinyl-N-Oxid, Indazolyl-N-Oxid, Chinolyl-N-Oxid, Isochinolyl-N-Oxid, Phthalazinyl-N-Oxid, Chinoxalinyl-N-Oxid, Chinazolinyl-N-Oxid, Cinnolinyl-N-Oxid],
das unsubstituiert oder substituiert ist mit 1 - 2 Substituenten ausgewählt aus der Gruppe bestehend aus F, Cl, CF₃, CH₃, Methoxy, Hydroxy, Amino, Methylamino und Dimethylamino;
a Null oder 1;
R(11), R(12)
Wasserstoff oder Methyl;
und der jeweils andere Substituent R(2) und R(3)
unabhängig voneinander (C₁-C₄)-Alkyl, Wasserstoff, F, Cl, Br oder 1; R(4) und R(5)
unabhängig voneinander Wasserstoff, Methyl, F, Cl, -OR(32),
-NR(33)R(34) oder CF₃;
R(32), R(33) und R(34)
unabhängig voneinander Wasserstoff oder Methyl;
sowie deren pharmazeutisch verträgliche Salze.

2. Verbindungen der Formel I nach Anspruch 1, in der bedeuten:
R(1) (C₁-C₄)-Alkyl, (C₂-C₄)-Alkenyl oder -CₘH₂ₘR(14);
m Null, 1 oder 2;
R(14) (C₅-C₆)-Cycloalkyl oder Phenyl,
welches nicht substituiert oder substituiert ist mit 1 - 2 Substituenten ausgewählt aus der Gruppe bestehend aus F, Cl, CF₃, Methyl, Methoxy und NR(15)R(16);
R(15) und R(16)
Wasserstoff oder CH₃;
oder
R(1) Wasserstoff, F, Cl, Br, I, -C≡N, R(22)-SO₂, R(23)R(24)N-CO, R(25)-CO-oder R(26)R(27)N-SO₂-;
R(22), R(23), R(25) und R(26)
unabhängig voneinander Methyl oder CF₃;
oder
R(23), R(25) und R(26)
Wasserstoff;
R(24) und R(27)
unabhängig voneinander Wasserstoff oder Methyl;
oder
R(1) OR(35) oder NR(35)R(36);
R(35) und R(36)
unabhängig voneinander Wasserstoff, (C₁-C₄)-Alkyl;
oder
R(35) und R(36)
gemeinsam 4 - 5 Methylengruppen, von denen eine CH₂-Gruppe durch Sauerstoff, S, NH, N-CH₃ oder N-Benzyl ersetzt sein kann,
einer der Substituenten R(2) und R(3)
Imidazolyl-N-Oxid, Pyrazolyl-N-Oxid, Triazolyl-N-Oxid, Tetrazolyl-N-Oxid, Oxazolyl-N-Oxid, Isoxazolyl-N-Oxid, Thiazolyl-N-Oxid, Isothiazolyl-N-Oxid, Pyridyl-N-Oxid, Pyrazinyl-N-Oxid, Pyrimidinyl-N-Oxid, Pyridazinyl-N-Oxid, Indazolyl-N-Oxid, Chinolyl-N-Oxid, Isochinolyl-N-Oxid, Phthalazinyl-N-Oxid, Chinoxalinyl-N-Oxid, Chinazolinyl-N-Oxid, Cinnolinyl-N-Oxid,
das über C oder N verknüpft ist und das unsubstituiert oder substituiert ist mit 1 - 2 Substituenten ausgewählt aus der Gruppe bestehend aus F, Cl, CF₃, CH₃, Methoxy, Hydroxy und Dimethylamino;
oder
einer der Substituenten R(2) und R(3)
-SR(10), -OR(10), -NR(10)R(11) oder -CR(10)R(11)R(12);
R(10) Imidazolyl-N-Oxid, Pyrazolyl-N-Oxid, Triazolyl-N-Oxid, Tetrazolyl-N-Oxid, Oxazolyl-N-Oxid, Isoxazolyl-N-Oxid, Thiazolyl-N-Oxid, Isothiazolyl-N-Oxid, Pyridyl-N-Oxid, Pyrazinyl-N-Oxid, Pyrimidinyl-N-Oxid, Pyridazinyl-N-Oxid, Indazolyl-N-Oxid, Chinolyl-N-Oxid, Isochinolyl-N-Oxid, Phthalazinyl-N-Oxid, Chinoxalinyl-N-Oxid, Chinazolinyl-N-Oxid, Cinnolinyl-N-Oxid,
das unsubstituiert oder substituiert ist mit 1 - 2 Substituenten ausgewählt aus der Gruppe bestehend aus F, Cl, CF₃, CH₃, Methoxy, Hydroxy und Dimethylamino;
R(11) und R(12)
unabhängig voneinander Wasserstoff oder Methyl;
und der jeweils andere Substituent R(2) und R(3)
unabhängig voneinander (C₁-C₄)-Alkyl, Wasserstoff, F, Cl, Br oder I;
R(4) und R(5)
unabhängig voneinander Wasserstoff, Methyl, F, Cl, -OR(32), - NR(33)R(34) oder CF₃;
R(32), R(33) und R(34)
unabhängig voneinander Wasserstoff oder Methyl.

3. Verfahren zur Herstellung einer Verbindung der Formel I nach Anspruch 1, **dadurch gekennzeichnet, daß** man
eine Verbindung der Formel II 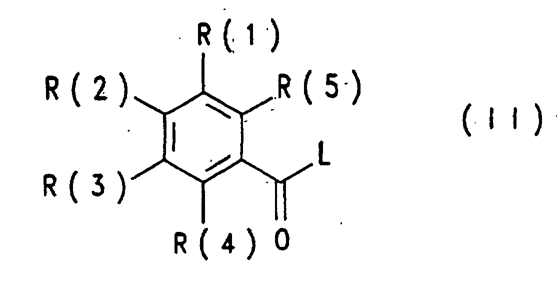 in welcher R(1) bis R(5) die in Anspruch 1 angegebenen Bedeutungen haben, mit Guanidin umsetzt, worin L für eine leicht nucleophil substituierbare Fluchtgruppe steht.

4. Verwendung einer Verbindung I nach Anspruch 1 zur Herstellung eines Medikaments zur Behandlung von Arrhythmien.

5. Verwendung einer Verbindung I nach Anspruch 1 zur Herstellung eines Medikaments zur Behandlung oder Prophylaxe des Herzinfarkts.

6. Verwendung einer Verbindung I nach Anspruch 1 zur Herstellung eines Medikaments zur Behandlung oder Prophylaxe der Angina Pectoris.

7. Verwendung einer Verbindung I nach Anspruch 1 zur Herstellung eines Medikaments zur Behandlung oder Prophylaxe von ischämischen Zuständen des Herzens.

8. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

9. Verwendung einer Verbindung I nach Anspruch 1 zur Herstellung eines Medikaments zur Behandlung oder Prophylaxe von ischämischen Zuständen peripherer Organe und Gliedmaßen.

10. Verwendung einer Verbindung 1 nach Anspruch 1 zur Herstellung eines Medikaments zur Behandlung von Schockzuständen.

11. Verwendung einer Verbindung I nach Anspruch 1 zur Herstellung eines Medikaments zum Einsatz bei chirurgischen Operationen und Organtransplantationen.

12. Verwendung einer Verbindung I nach Anspruch 1 zur Herstellung eines Medikaments zur Konservierung und Lagerung von Transplantaten für chirurgische Maßnahmen.

13. Verwendung einer Verbindung I nach Anspruch 1 zur Herstellung eines Medikaments zur Behandlung von Krankheiten, bei denen die Zellproliferation eine primäre oder sekundäre Ursache darstellt.

14. Heilmittel, enthaltend eine wirksame Menge einer Verbindung I nach Anspruch 1.

## Claims

1. A benzoylguanidine of the formula I 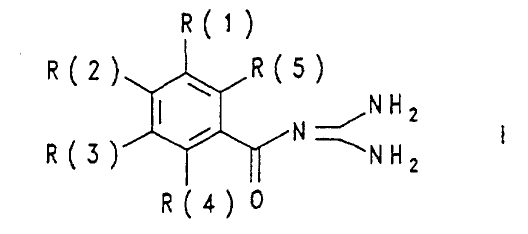 in which:
R(1) is (C₁-C₄)-alkyl, (C₂-C₄)-alkenyl or -CₘH₂ₘR(14),
m is zero, 1 or 2;
R(14) is (C₅-C₆)-cycloalkyl or phenyl,
which is unsubstituted or substituted by 1 - 2 substituents chosen from the group consisting of F, Cl, CF₃, methyl, methoxy and NR(15)R(16);
R(15) and R(16) are
hydrogen or CH₃;
or
R(1) is hydrogen, F, Cl, Br, I, -C=N, R(22)-SO₂, R(23)-R(24)N-CO, R(25)-CO- or R(26)R(27)N-SO₂-;
R(22), R(23), R(25) and R(26)
independently of one another are (C₁-C₄)-alkyl, (C₂-C₄)-alkenyl, -CₙH₂ₙ-R(29) or CF₃;
n is zero, 1 or 2;
R(29) is (C₅-C₆)-cycloalkyl or phenyl,
which is unsubstituted or substituted by 1 - 2 substituents chosen from the group consisting of F, Cl, CF₃, methyl, methoxy and NR(30)R(31);
R(30) and R(31) are
hydrogen or methyl;
or
R(23), R(25) and R(26) are
hydrogen;
R(24) and R(27)
independently of one another are hydrogen or methyl;
or R(23) and (R24), and R(26) and R(27)
together are 4 or 5 methylene groups, one CH₂ group of which can be replaced by oxygen, S, NH, N-CH₃ or N-benzyl;
or
R(1) is OR(35) or NR(35)R(36);
R(35) and R(36)
independently of one another are hydrogen or (C₁-C₄)-alkyl;
or R(35) and R(36)
together are 4 - 5 methylene groups, one CH₂ group of which can be replaced by oxygen, S, NH, N-CH₃ or N-benzyl;
one of the substituents R(2) and R(3) is
imidazolyl-N-oxide, pyrazolyl-N-oxide, triazolyl-N-oxide, tetrazolyl-N-oxide, oxazolyl-N-oxide, isoxazolyl-N-oxide, thiazolyl-N-oxide, isothiazolyl-N-oxide, pyridyl-N-oxide, pyrazinyl-N-oxide, pyrimidinyl-N-oxide, pyridazinyl-N-oxide, indazolyl-N-oxide, quinolyl-N-oxide, isoquinolyl-N-oxide, phthalazinyl-N-oxide, quinoxalinyl-N-oxide, quinazolinyl-N-oxide and quinnolinyl-N-oxide,
which is linked via C or N and is unsubstituted or substituted by 1 - 2 substituents chosen from the group consisting of F, Cl, CF₃, CH₃, methoxy, hydroxy, amino, methylamino and dimethylamino;
or one of the substituents R(2) and R(3) is
-SR(10), -OR(10), -NR(10)R(11) or -CR(10)R(11)-R(12);
R(10) is -CₐH₂ₐ-[imidazolyl-N-oxide, pyrazolyl-N-oxide, triazolyl-N-oxide, tetrazolyl-N-oxide, oxazolyl-N-oxide, isoxazolyl-N-oxide, thiazolyl-N-oxide, isothiazolyl-N-oxide, pyridyl-N-oxide, pyrazinyl-N-oxide, pyrimidinyl-N-oxide, pyridazinyl-N-oxide, indazolyl-N-oxide, quinolyl-N-oxide, isoquinolyl-N-oxide, phthalazinyl-N-oxide, quinoxalinyl-N-oxide, quinazolinyl-N-oxide and quinnolinyl-N-oxide],
which is unsubstituted or substituted by 1 - 2 substituents chosen from the group consisting of F, Cl, CF₃, CH₃, methoxy, hydroxy, amino, methylamino and dimethylamino;
a is zero or 1;
R(11) and R(12) are
hydrogen or methyl;
and the other particular substituent R(2) or R(3) is (C₁-C₄)-alkyl, hydrogen, F, Cl, Br or I;
R(4) and R(5)
independently of one another are hydrogen, methyl, F, Cl, -OR(32), -NR(33)R(34) or CF₃;
R(32), R(33) and R(34)
independently of one another are hydrogen or methyl,
or a pharmaceutically tolerated salt thereof.

2. A compound of the formula I as claimed in claim 1, in which:
R(1) is (C₁-C₄)-alkyl, (C₂-C₄)-alkenyl or -CₘH₂ₘR(14);
m is zero, 1 or 2;
R(14) is (C₅-C₆)-cycloalkyl or phenyl,
which is unsubstituted or substituted by 1 - 2 substituents chosen from the group consisting of F, Cl, CF₃, methyl, methoxy and NR(15)R(16);
R(15) and R(16) are
hydrogen or CH₃;
or
R(1) is hydrogen, F, Cl, Br, I, -C≡N, R(22)-SO₂,
R(23)R(24)N-CO, R(25)-CO- or R(26)R(27)N-SO₂- ;
R(22), R(23), R(25) and R(26)
independently of one another are methyl or CF₃;
or
R(23), R(25) and R(26) are
hydrogen;
R(24) and R(27)
independently of one another are hydrogen or methyl;
or
R(1) is OR(35) or NR(35)R(36);
R(35) and R(36)
independently of one another are hydrogen or (C₁-C₄)-alkyl;
or
R(35) and R(36)
together are 4 - 5 methylene groups, one CH₂ group of which can be replaced by oxygen, S, NH, N-CH₃ or N-benzyl,
one of the substituents R(2) and R(3) is
imidazolyl-N-oxide, pyrazolyl-N-oxide, triazolyl-N-oxide, tetrazolyl-N-oxide, oxazolyl-N-oxide, isoxazolyl-N-oxide, thiazolyl-N-oxide, isothiazolyl-N-oxide, pyridyl-N-oxide, pyrazinyl-N-oxide, pyrimidinyl-N-oxide, pyridazinyl-N-oxide, indazolyl-N-oxide, quinolyl-N-oxide, isoquinolyl-N-oxide, phthalazinyl-N-oxide, quinoxalinyl-N-oxide, quinazolinyl-N-oxide and quinnolinyl-N-oxide,
which is linked via C or N and is unsubstituted or substituted by 1 - 2 substituents chosen from the group consisting of F, Cl, CF₃, CH₃, methoxy, hydroxy and dimethylamino;
or
one of the substituents R(2) and R(3) is
-SR(10), -OR(10), -NR(10)R(11) or -CR(10)R(11)-R(12);
R(10) is imidazolyl-N-oxide, pyrazolyl-N-oxide, triazolyl-N-oxide, tetrazolyl-N-oxide, oxazolyl-N-oxide, isoxazolyl-N-oxide, thiazolyl-N-oxide, isothiazolyl-N-oxide, pyridyl-N-oxide, pyrazinyl-N-oxide, pyrimidinyl-N-oxide, pyridazinyl-N-oxide, indazolyl-N-oxide, quinolyl-N-oxide, isoquinolyl-N-oxide, phthalazinyl-N-oxide, quinoxalinyl-N-oxide, quinazolinyl-N-oxide and quinnolinyl-N-oxide,
which is unsubstituted or substituted by 1 - 2 substituents chosen from the group consisting of F, Cl, CF₃, CH₃, methoxy, hydroxy and dimethylamino;
R(11) and R(12) are
hydrogen or methyl;
and the other particular substituent R(2) or R(3) is independently of one another (C₁-C₄)-alkyl, hydrogen, F, Cl, Br or I;
R(4) and R(5)
independently of one another are hydrogen, methyl, F, Cl, -OR(32), -NR(33)R(34) or CF₃;
R(32), R(33) and R(34)
independently of one another are hydrogen or methyl.

3. A process for the preparation of a compound of the formula I as claimed in claim 1, which comprises reacting a compound of the formula II 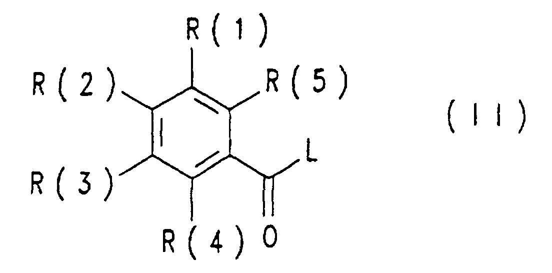 in which R(1) to R(5) have the meanings given in claim 1,
with guanidine, in which L is a leaving group which can easily be substituted nucleophilically.

4. The use of a compound I as claimed in claim 1 for the preparation of a medicament for the treatment of arrhythmias.

5. The use of a compound I as claimed in claim 1 for the preparation of a medicament for the treatment or prophylaxis of cardiac infarction.

6. The use of a compound I as claimed in claim 1 for the preparation of a medicament for the treatment or prophylaxis of angina pectoris.

7. The use of a compound I as claimed in claim 1 for the preparation of a medicament for the treatment or prophylaxis of ischemic states of the heart.

8. The use of a compound I as claimed in claim 1 for the preparation of a medicament for the treatment or prophylaxis of ischemic states of the peripheral and central nervous system and of apoplexy.

9. The use of a compound I as claimed in claim 1 for the preparation of a medicament for the treatment or prophylaxis of ischemic states of peripheral organs and limbs.

10. The use of a compound I as claimed in claim 1 for the preparation of a medicament for the treatment of states of shock.

11. The use of a compound I as claimed in claim 1 for the preparation of a medicament for use during surgical operations and organ transplants.

12. The use of a compound I as claimed in claim 1 for the preparation of a medicament for preserving and storing transplants for surgical measures.

13. The use of a compound I as claimed in claim 1 for the preparation of a medicament for the treatment of diseases with which cell proliferation is a primary or secondary cause.

14. A medicine comprising an active amount of a compound I as claimed in claim 1.

## Revendications

1. Benzoylaguanidine de formule I 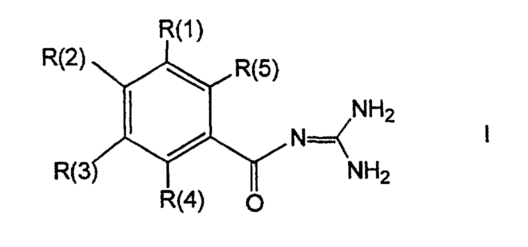 dans laquelle :
R(1) est un radical alkyle (en C₁-C₄), alcényle (en C₂-C₄) ou -CₘH₂ₘR(14),
m vaut zéro, 1 ou 2 ;
R(14) est un radical cycloalkyle (en C₅-C₆) ou phényle,
qui est non substitué ou substitué par 1 - 2 substituants choisis dans le groupe constitué par F, Cl, CF₃, un radical méthyle, méthoxy et NR(15)R(16) ; R(15) et R(16)
sont l'hydrogène ou CH₃;
ou
R(1) est l'hydrogène, F, Cl, Br, I, -C≡N, R(22)-SO₂, R(23)R(24)N-CO ,
R(25)-CO- ou R(26)R(27)N-SO₂-;
R(22), R(23), R(25) et R(26)
sont indépendamment l'un de l'autre un radical alkyle (en C₁-C₄), alcényle (en C₂-C₄) ou -CₙH₂ₙR(29), ou CF₃;
n vaut zéro, 1 ou 2 ;
R(29) estun radical cycloalkyle (en C₅-C₆) ou phényle,
qui est non substitué ou substitué par 1 - 2 substituants choisis dans le groupe constitué par F, Cl, CF₃, un radical méthyle, méthoxy et NR(30)R(31) ;
R(30) et R(31)
sont l'hydrogène ou un radical méthyle ;
ou
R(23), R(25) et R(26)
sont l'hydrogène ;
R(24) et R(27)
sont indépendamment l'un de l'autre l'hydrogène ou un radical méthyle ;
ou R(23) et R(24) ainsi que R(26) et R(27)
sont ensemble 4 ou 5 radicaux méthylène dont un radical CH₂ peut être remplacé par l'oxygène, S, un radical NH, N-CH₃ ou N-benzyle ;
ou
R(1) est OR(35) ou NR(35)R(36) ;
R(35) et R(36)
sont indépendamment l'un de l'autre l'hydrogène, un radical alkyle en (C₁-C₄) ;
ou R(35) et R(36)
sont ensemble 4 - 5 groupes méthylène, dont un radical CH₂ peut être remplacé par l'oxygène, S, un radical NH, N-CH₃ ou N-benzyle ;
un des substituants R(2) et R(3)
est un radical imidazolyl-N-oxyde, pyrazolyl-N-oxyde, triazolyl-N-oxyde, tétrazolyl-N-oxyde, oxazolyl-N-oxyde, isoxazolyl-N-oxyde, thiazolyl-N-oxyde, isothiazolyl-N-oxyde, pyridyl-N-oxyde, pyrazinyl-N-oxyde, pyrimidinyl-N-oxyde, pyridazinyl-N-oxyde, indazolyl-N-oxyde, quinolyl-N-oxyde, isoquinolyl-N-oxyde, phtalazinyl-N-oxyde, quinoxalinyl-N-oxyde, quinazolinyl-N-oxyde, cinnolinyl-N-oxyde,
qui est rattaché sur C ou N et qui est non substitué ou substitué par 1 - 2 substituants choisis dans le groupe constitué par F, Cl, CF₃, CH₃, un radical méthoxy, hydroxy, amino, méthylamino et diméthylamino ;
ou un des substituants R(2) et R(3)
est -SR(10), -OR(10), -NR(10)R(11) ou -CR(10)R(11)R(12); R(10) est un radical -CₐH₂ₐ-[imidazolyl-N-oxyde, pyrazolyl-N-oxyde, triazolyl-N-oxyde, tétrazolyl-N-oxyde, oxazolyl-N-oxyde, isoxazolyl-N-oxyde, thiazolyl-N-oxyde, isothiazolyl-N-oxyde, pyridyl-N-oxyde, pyrazinyl-N-oxyde, pyrimidinyl-N-oxyde, pyridazinyl-N-oxyde, indazolyl-N-oxyde, quinolyl-N-oxyde, isoquinolyl-N-oxyde, phtalazinyl-N-oxyde, quinoxalinyl-N-oxyde, quinazolinyl-N-oxyde, cinnolinyl-N-oxyde],
qui est non substitué ou substitué par 1 - 2 substituants choisis dans le groupe constitué par F, Cl, CF₃, CH₃, un radical méthoxy, hydroxy, amino, méthylamino et diméthylamino ;
a vaut zéro ou 1 ;
R(11), R(12)
sont l'hydrogène ou un radical méthyle ;
et chacun de l'autre substituant R(2) et R(3)
est indépendamment l'un de l'autre un radical alkyle (en C₁-C₄), l'hydrogène, F, Cl, Br ou I ;
R(4) et R(5)
sont indépendamment l'un de l'autre l'hydrogène, un radical méthyle, F, Cl, -OR(32), -NR(33)R(34) ou CF₃;
R(32), R(33) et R(34) sont indépendamment l'un de l'autre l'hydrogène ou un radical méthyle ;
ainsi que leurs sels acceptables du point de vue pharmaceutique.

2. Composés de formule I selon la revendication 1, dans laquelle :
(R1) est un radical alkyle (en C₁-C₄), alcényle (en C₂-C₄) ou -CₘH₂ₘR(14),
m vaut zéro, 1 ou 2 ;
R(14) est un radical cycloalkyle (en C₅-C₆) ou phényle,
qui est non substitué ou substitué par 1 - 2 substituants choisis dans le groupe constitué par F, Cl, CF₃, un radical méthyle, méthoxy et NR(15)R(16) ; R(15) et R(16)
sont l'hydrogène ou CH₃ ;
ou
R(1) est l'hydrogène, F, Cl, Br, I, -C≡N, R(22)-SO₂, R(23)R(24)N-CO,
R(25)-CO- ou R(26)R(27)N-SO₂- ;
R(22), R(23), R(25) et R(26)
sont indépendamment l'un de l'autre un radical méthyle ou CF3 ;
ou
R(23), R(25) et R(26)
sont l'hydrogène ;
R(24) et R(27)
sont indépendamment l'un de l'autre l'hydrogène ou un radical méthyle ;
ou
R(1) est OR(35) ou NR(35)R(36) ;
R(35) et R(36)
sont indépendamment l'un de l'autre l'hydrogène, un radical alkyle (en C₁-C₄);
ou
R(35) et R(36)
sont ensemble 4 - 5 radicaux méthylène dont un radical CH₂ peut être remplacé par l'oxygène, S, un radical NH, N-CH₃ ou N-benzyle ;
un des substituants R(2) et R(3)
est un radical imidazolyl-N-oxyde, pyrazolyl-N-oxyde, triazolyl-N-oxyde, tétrazolyl-N-oxyde, oxazolyl-N-oxyde, isoxazolyl-N-oxyde, thiazolyl-N-oxyde, isothiazolyl-N-oxyde, pyridyl-N-oxyde, pyrazinyl-N-oxyde, pyrimidinyl-N-oxyde, pyridazinyl-N-oxyde, indazolyl-N-oxyde, quinolyl-N-oxyde, isoquinolyl-N-oxyde, phtalazinyl-N-oxyde, quinoxalinyl-N-oxyde, quinazolinyl-N-oxyde, cinnolinyl-N-oxyde,
qui est rattaché sur C ou N et qui est non substitué ou substitué par 1 - 2 substituants choisis dans le groupe constitué par F, Cl, CF₃, CH₃, un radical méthoxy, hydroxy et diméthylamino ;
ou un des substituants R(2) et R(3)
est -SR(10), -OR(10), -NR(10)R(11) ou -CR(10)R(11)R(12) ;
R(10) est un radical imidazolyl-N-oxyde, pyrazolyl-N-oxyde, triazolyl-N-oxyde, tétrazolyl-N-oxyde, oxazolyl-N-oxyde, isoxazolyl-N-oxyde, thiazolyl-N-oxyde, isothiazolyl-N-oxyde, pyridyl-N-oxyde, pyrazinyl-N-oxyde, pyrimidinyl-N-oxyde, pyridazinyl-N-oxyde, indazolyl-N-oxyde, quinolyl-N-oxyde, isoquinolyl-N-oxyde, phtalazinyl-N-oxyde, quinoxalinyl-N-oxyde, quinazolinyl-N-oxyde, cinnolinyl-N-oxyde,
qui est non substitué ou substitué par 1 - 2 substituants choisis dans le groupe constitué par F, Cl, CF₃, CH₃, un radical méthoxy, hydroxy et diméthylamino ;
R(11), R(12)
sont indépendamment l'un de l'autre l'hydrogène ou un radical méthyle ;
et chacun de l'autre substituant R(2) et R(3)
est indépendamment l'un de l'autre un radical alkyle (en C₁-C₄), l'hydrogène, F, Cl, Br ou I ;
R(4) et R(5)
sont indépendamment l'un de l'autre l'hydrogène, un radical méthyle, F, Cl, -OR(32), -NR(33)R(34) ou CF₃;
R(32), R(33) et R(34)
sont indépendamment l'un de l'autre l'hydrogène ou un radical méthyle.

3. Procédé de préparation d'un composé de formule I selon la revendication 1, **caractérisé en ce qu'**on fait réagir avec la guanidine un composé de formule II 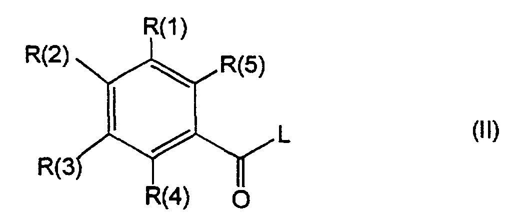 dans laquelle R(1) à R(5) ont les significations données dans la revendication 1, L étant un groupe partant substituable par un groupe légèrement nucléophile.

4. Utilisation d'un composé de formule I tel que revendiqué dans la revendication 1 pour la préparation d'un médicament pour le traitement des arythmies.

5. Utilisation d'un composé de formule I tel que revendiqué dans la revendication 1 pour la préparation d'un médicament pour le traitement ou la prophylaxie de l'infarctus du myocarde.

6. Utilisation d'un composé de formule I tel que revendiqué dans la revendication 1 pour la préparation d'un médicament pour le traitement ou la prophylaxie de l'angine de poitrine.

7. Utilisation d'un composé de formule I tel que revendiqué dans la revendication 1 pour la préparation d'un médicament pour le traitement ou la prophylaxie des états ischémiques du coeur.

8. Utilisation d'un composé de formule I tel que revendiqué dans la revendication 1 pour la préparation d'un médicament pour le traitement ou la prophylaxie des états ischémiques du système nerveux périphérique et central et de l'apoplexie.

9. Utilisation d'un composé de formule I tel que revendiqué dans la revendication 1 pour la préparation d'un médicament pour le traitement ou la prophylaxie des états ischémiques des organes périphériques et des extrémités.

10. Utilisation d'un composé de formule I tel que revendiqué dans la revendication 1 pour la préparation d'un médicament pour le traitement d'états de chocs.

11. Utilisation d'un composé de formule I tel que revendiqué dans la revendication 1 pour la préparation d'un médicament pour l'emploi dans des opérations chirurgicales et des transplantations d'organes.

12. Utilisation d'un composé de formule I tel que revendiqué dans la revendication 1 pour la préparation d'un médicament pour la conservation et le stockage d'organes à transplanter pour des opérations chirurgicales.

13. Utilisation d'un composé de formule I tel que revendiqué dans la revendication 1 pour la préparation d'un médicament pour le traitement de maladies pour lesquelles une prolifération cellulaire constitue une cause primaire ou secondaire.

14. Médicament contenant une quantité efficace d'un composé I selon la revendication 1.
